(19) Europäisches Patentamt
European Patent Office
Office européen des brevets

(11) Publication number : **0 452 384 B1**

(12) # EUROPEAN PATENT SPECIFICATION

(45) Date of publication of patent specification : **06.10.93 Bulletin 93/40**

(51) Int. Cl.$^5$ : **A61K 31/445,** A61K 9/12, A61K 9/72

(21) Application number : **90901641.2**

(22) Date of filing : **04.01.90**

(86) International application number : **PCT/GB90/00015**

(87) International publication number : **WO 90/07333 12.07.90 Gazette 90/16**

(54) **FENTANYL CONTAINING AEROSOL COMPOSITIONS.**

(30) Priority : **06.01.89 GB 8900267**

(43) Date of publication of application : **23.10.91 Bulletin 91/43**

(45) Publication of the grant of the patent : **06.10.93 Bulletin 93/40**

(84) Designated Contracting States : **AT BE CH DE DK ES FR GB IT LI LU NL SE**

(56) References cited :
**DE-A- 3 602 370**
**Remington's Pharmaceutical Sciences, 5th ed.(1975), Mack Publishing Company, p. 1044**

(73) Proprietor : **RIKER LABORATORIES, INC.**
**19901 Nordhoff Street**
**Northridge California (US)**

(72) Inventor : **PUREWAL, Tarlochan, Singh 196**
**Radford Road**
**Leamington Spa**
**Warwickshire CV31 1LQ (GB)**
Inventor : **WILKINSON, Anthony**
**9 Woodlands Drive Loughborough**
**Leicestershire LE11 3LR (GB)**
Inventor : **LAMBERT, Alison, Lesley**
**1 Goldgarth Grimsby**
**South Humberside DN32 8QS (GB)**
Inventor : **SMITH, David, Keith**
**8 Springfield Close**
**Loughborough LE11 3PT (GB)**
Inventor : **DONNELL, David Highthorne**
**Cottage Wide Lane**
**Wymeswold**
**Leicestershire LE12 6SE (GB)**
Inventor : **KUEPPER, Anton**
**Jupiterstrasse 13**
**D-4044 Kaarst 1 (DE)**

(74) Representative : **Bowman, Paul Alan et al**
**LLOYD WISE, TREGEAR & CO. Norman House**
**105-109 Strand**
**London WC2R OAE (GB)**

EP 0 452 384 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Jouve, 18, rue Saint-Denis, 75001 PARIS

## Description

This invention relates to analgesic formulations and in particular to analgesic formulations comprising fentanyl suitable for administration by inhalation.

Narcotic analgesics are used to relieve moderate to severe pain, particularly of a visceral origin. The narcotic analgesics are generally administered by subcutaneous, intra-muscular or intravenous injection, or orally in the form of elixirs, tablets (optionally sublingual) and capsules, or by rectal administration in the form of suppositories. In the case of patients who are hospitalised narcotic analgesics are often administered in the form of saline drips.

Since the metered dose pressurised inhaler was introduced in the mid 1950's, inhalation has become the most widely used route for delivering bronchodilator drugs and steroids to the airways of asthmatic patients. Compared with oral administration of bronchodilators, inhalation offers a rapid onset of action and a low instance of systemic side effects. More recently, inhalation from a pressurised inhaler has been a route selected for the administration of other drugs, e.g., ergotamine, which are not primarily concerned with treatment of a bronchial malady.

Various publications, e.g., British Patents Nos. 830426, 837465, 994734 and 2125426; European Patent Nos. 0162239 and WO86/04233 which relate to self-propelling pharmaceutical compositions for administration from pressurised inhalers disclose the possibility of employing an analgesic such as morphine, diamorphine and buprenorphine hydrochloride in such formulations although there is no disclosure of any specific formulations containing such analgesics nor any indication of their efficiency when administered by inhalation.

DE3602370 discloses the administration of analgesics by inhalation for the treatment of pain. The specification discloses the administration of analgesics by insufflation and the use of inhalation solutions. Tests were conducted on patients for post-operative treatment of pain and the results indicate satisfactory analgesia may be obtained with morphine and other analgesics including Buprenorphin, Tramal, Fentanyl and AL-fentanyl. Suitable administration doses are provided for each drug. There is no disclosure of the administration of the analgesics by use of a self-propelling aerosol composition.

It has now been found that morphine and diamorphine hydrochloride are considerably less potent when administered by inhalation using self-propelling aerosol compositions than might have been expected from the known intravenous and other dosing data. It has also been found that fentanyl and in particular fentanyl citrate exhibits a potent, quick acting effect when administered by inhalation from a self-propelling aerosol formulation.

Therefore according to the present invention there is provided an aerosol formulation comprising fentanyl or a physiologically acceptable derivative thereof dispersed or dissolved in an aerosol propellant.

The invention also provides a pressurised aerosol inhaler comprising a container, containing an aerosol formulation as defined above, and a valve capable of dispensing metered doses of the formulation. The pressurised aerosol preferably incorporates the means to control the dosing frequency from the valve such that not more than a predetermined maximum number of doses may be dispensed within a set period of time. Such a pressurised inhaler allows the maximum dosage frequency available to the patient to be pre-set, whilst insuring the patient cannot receive an overdose. The inhaler provides the benefits of on-demand dosing for the patient with dosage control, and may be used both in hospitals and homes without requiring medical personnel to administer each dose.

The formulations used in the invention contain fentanyl or a derivative thereof either in solution or suspension in the aerosol propellant system, optionally in the presence of a cosolvent. The solvent for fentanyl will generally be present in an amount in the range 5 to 25% by weight of the composition. The compositions may additionally comprise one or more surface active agents, for example oleic acids, complex esters and ester-ethers, e.g., sorbitan trioleate, commercially available under the registered trade mark SPAN® 85, lecithins, commercially available under the registered trade mark EPIKURON® 200, and fluorinated surfactants. The weight ratio of surface active agent to fentanyl is generally in the range 1 : 100 to 10 : 1. The concentration of fentanyl will generally be from 0.05 to 5.00%, preferably 0.1 to 1.0%, by weight based on the total composition.

A wide range of propellants may be used in the aerosol formulations of the invention including:

| | |
|---|---|
| Propellant 11 | trichloromonofluoromethane |
| Propellant 12 | dichlorodifluoromethane |
| Propellant 13 | monochlorotrifluoromethane |
| Propellant 21 | dichloromonofluoromethane |
| Propellant 22 | monochlorodifluoromethane |
| Propellant 113 | trichlorotrifluoroethane |
| Propellant 114 | dichlorotetrafluoroethane |
| Propellant 115 | monochloropentafluoroethane |
| Propellant 134a | 1,1,1,2-tetrafluoroethane |

Propellant 500 azeotrope of dichlorodifluoromethane and 1,1-difluoroethane

In addition to chlorofluorocarbon aerosol propellants the formulations may contain other propellants, or example, DME (dimethylether), hydrocarbons and perfluorocarbons.

One preferred propellant system is disclosed in our co-pending British Patent Application No. 8828477.3 and comprises 1,1,1,2-tetrafluoroethane, a surface active agent and at least one compound having a higher polarity than 1,1,1,2-tetrafluoroethane. Suitable compounds having a higher polarity than 1,1,1,2-tetrafluoroethane include alcohols, such as ethyl alcohol, isopropyl alcohol, propylene glycol, hydrocarbons such as propane, butane, isobutane, pentane, isopentane, neopentane, and mixtures thereof. The 1,1,1,2-tetrafluoroethane preferably comprises at least 50% by weight of the formulation, preferably from 60 to 95% by weight of the formulation. The weight ratio of 1,1,1,2-tetrafluoroethane to the compound of higher polarity is generally in the range 50 : 50 to 99 : 1, preferably 70 : 30 to 98: 2, more preferably 85 : 15 to 95 : 5.

In an alternative system the fentanyl or derivative thereof in the form of a finely divided solid is coated with a dry coating of a perfluorinated surface-active dispersing agent and thereafter mixed with an aerosol propellant. Such systems are disclosed generally in U.S. Patent No. 4,352,789. The preferred propellant for such formulation is 1,1,1,2-tetrafluoroethane, preferably with an adjuvant having a polarity equal to or lower than the polarity of 1,1,1,2-tetrafluoroethane.

Suitable adjuvants having a polarity equal to or lower than Propellant 134a include perfluorinated organic compounds such as perfluorinated alkanes and cycloalkanes. Specific examples of adjuvants include perfluoropropane, perfluorobutane, perfluorocyclobutane, perfluoropentane, perfluorohexane, perfluorotributylamine, perfluoromethylcyclohexane and perfluorodecalin. Such compositions generally comprise from 0.001 to 20% by weight of finely-divided solid fentanyl coated with a perfluorinated surface-active dispersing agent which constitutes at least 0.001%, normally 0.001 to 50%, preferably 0.001 to 20% by weight of the coated solid material, and suspended in an aerosol propellant comprising 1,1,1,2-tetrafluoroethane and an adjuvant having a polarity equal to or lower than that of 1,1,1,2-tetrafluoroethane.

The perfluorinated surface-active dispersing agents (hereinafter referred to as "perfluorinated surfactants" or "surfactants") are substantially insoluble in the propellant. This insolubility is due to the relatively ionic character of one end of the surfactant molecule. This ionic group is compatible with the solid powdered material and enables the surfactant to wet the solid material. Although the perfluorinated surfactant is insoluble in the propellant, when coated on the solid material, the outermost perfluorinated groups of the surfactant allow the solid coated material to be dispersed in the propellant due to the compatibility between the perfluorinated groups and the propellant.

Perfluorinated surfactants most useful in the compositions of the present invention include perfluorinated alcohol phosphate esters and their salts; perfluorinated sulfonamide alcohol phosphate esters and their salts; perfluorinated alkyl sulfonamide alkylene quaternary ammonium salts; N,N-(carboxyl-up to $C_4$ substituted alkyl) perfluorinated alkyl sulfonamides and their salts; and mixtures thereof. By "perfluorinated" it is meant that the surfactant contains at least one perfluorinated alkyl group. Particularly preferred perfluorinated alcohol phosphate esters are the free acids of the diethanolamine salts of mono- and bis(1H,1H,2H,2H-perfluoroalkyl) phosphates. The phosphate salts, available under the registered trade mark "Zonyl® RP" from E. I Dupont de Nemours and Company, Wilmington, Del., are converted to the corresponding free acids.

Preferred perfluorinated sulfonamide alcohol phosphate esters are described in U.S. Patent No. 3,094,547, and have the general formula:

$$[R_fSO_2N(R)R'O]_m\overset{\overset{\large O}{\|}}{P}(OH)_{3-m}$$

in which;

R is hydrogen or an alkyl group having from 1 to 12, preferably from 1 to 6, carbon atoms; R' is an alkylene bridging group containing 2 to 12 carbon atoms, preferably from 2 to 8 carbon atoms; $R_f$ is a perfluorinated radical selected from perfluoroaliphatic groups of general formula $C_nF_{2n+1}$ or perfluorocycloaliphatic groups of general formula $C_nF_{2n-1}$ in which; n is an integer from 1 to 18, preferably from 6 to 12, and m is an integer from 1 to 3.

Although the mono-, di- and triesters are useful, the diester is most readily available commercially. Particularly preferred perfluorinated sulfanomide alcohol phosphate esters and salts of these include perfluoro-n-octyl-N-ethylsulfonamidoethyl phosphate, bis(perfluoro-n-octyl-N-ethylsulfonamidoethyl)phosphate, the ammonium salt of bis(perfluoro-n-octyl-N-ethyl-sulfonamidoethyl) phosphate, bis(perfluorodecyl-N-ethylsulfonamidoethyl) phosphate and bis(perfluorohexyl-N-ethylsulfonamidoethyl) phosphate. The above named

preferred surfactants are of particular use in medicinal aerosol compositions due to their non-irritating and non-toxic nature.

The preferred perfluorinated alkyl sulfonamide alkylene quaternary ammonium salt for use in the preparation of aerosol medicaments according to the present invention is N,N-dimethyl-N-decyl-N-(perfluoro-n-octylsulfonamidopropyl)ammonium bromide.

A preferred N,N-bis(carboxyl-up to $C_4$ substituted alkyl)perfluorinated alkyl sulfonamide for use with medicaments in aerosol compositions of the present invention is N,N-bis(4-carboxyl-n-butylperfluoro-n-octylsulfonamide).

The perfluorinated surfactant constitutes at least 0.001% and generally up to 50%, usually up to 20%, desirably between 0.01 and 5%, and preferably, for medicinal purposes, between 0.01 and 1% by weight of the solid material to be suspended. However, the minimum amount of perfluorinated surfactant required is dependent upon the concentration of solid material present. For best results, the concentration of perfluorinated surface-active agent is kept at a minimum as it may tend to increase the droplet size of the aerosol particles.

The particle size of the powder should desirably be no greater than 100μm diameter, since larger particles may tend to agglomerate, separate from the suspension and may clog the valve or orifice of the container. Preferably the particle size should be less than 25μm in diameter. Desirably the particle size of the finely-divided solid powder should for physiological reasons be less than 25μm and preferably less than 10μm in diameter. The particle size of the powder for inhalation therapy should preferably be in the range 2 to 10μm.

The finely-divided solid material may constitute up to 20% by weight of the total composition. Desirably it shall constitute up to 10%, generally up to 5% and preferably up to 3%, by weight of the total composition. The minimum concentration of the solid material is governed by its specific activity and in the case of highly active material can be as low as 0.001% by weight of the total composition although a concentration of 0.01% is preferred.

It is also possible to coat finely divided solid fentanyl or derivatives thereof with non-perfluorinated surface-active agents in similar manner and thereafter mix with a wide range of aerosol propellants. Propellant 134a is preferred because of its ozone friendly properties.

Other propellant systems which may be employed include mixtures of aerosol propellants. General concentration ranges for specific propellants which may be employed in admixtures are as follows:

| | |
|---|---|
| Propellant 11 | : 15 to 25% by weight |
| Propellant 12 | : 50 to 90% by weight |
| Propellant 22 | : 5 to 50% by weight |

The aerosol formulations of the invention are preferably used in a pressurised aerosol inhaler comprising a container and a valve capable of dispensing doses of the formulation, in which the inhaler additionally comprises means to control the dosing frequency from the valve.

The control means preferably comprises an electronic timing device associated with means to prevent actuation of the valve of the pressurised aerosol inhaler such that the inhaler may be disabled until the end of a controlled period of time during which a pre-set maximum number of doses have been dispensed. The electronic time control of the dosing frequency can take either of the following forms:

(i) The patient can take from 1 to 'N' doses at the start of the control period 'T'. The clock starts when the first dose is dispensed; the patient then has a shorter period 't' to take further doses up to 'N'. The device then locks out for the remainder of the control period 'T'.

(ii) The patient can take up to 'N' doses during the whole of the control period. The clock starts at the first dose in a new control period.

Option (ii) is the preferred dosage-control mode for most situations. The control period, 'T', and the maximum number of doses 'N' can be either factory pre-set or adjustable by medical or nursing staff. The inhaler device may have provision for internal adjustment of these control parameters. Alternatively, the controls may be positioned externally using one-way rotary switches which allow only increase of time 'T' and reduction of the maximum number of doses 'N' to prevent overdosage. The parameters may be set to ensure a sufficient time has elapsed for a dose to take effect before the patient may take a further dose.

A further control feature which may be incorporated in the device is a locking mechanism designed to effect permanent disablement of the device after the label-claim number of doses has been dispensed, thus allowing precise control of the number of doses available from each aerosol container.

A further feature which may be incorporated into the inhaler device is a liquid crystal display which may display a variety of information, for example:

(a) the balance of the label-claim number of doses remaining in the aerosol canister,

(b) the time remaining in the current control period, and,

(c) the number of available doses remaining in the current control period.

Suitable inhaler devices are shown in the accompanying drawings in which:

Figure 1 represents a diagram of an inhaler, and,

Figure 2 represents a block diagram of an electrical circuit for use in the inhaler of Figure 1.

Referring to Figure 1 of the accompanying drawings, the inhalation device comprises a housing 2 accommodating an aerosol container 4 having a dispensing valve 6. The aerosol container 4 is mounted vertically with the outlet valve 6 positioned within a nozzle block 8. The housing 2 has a mouthpiece 10 which may be adapted by the provision of a nasal adaptor if the medicament is to be administered via the nose. In use, the patient inhales via the mouthpiece 10, initiating movement of the container relative to the valve, causing a dose of medicament to be fired from the valve, through the nozzle block, into the mouthpiece, and thence into the lungs as the patient inhales.

In the inhaler shown in Figure 1, the start of inhalation is sensed by a precision-moulded triggering mechanism which responds to an inhalation flow rate of about 30 litres/minute. The triggering mechanism comprises a vane 20 associated with a locking device acting on the nozzle block 8, allowing actuation of the aerosol device only during inhalation. Examples of such arrangements are disclosed in European Patent No. 0147028B. Force is first applied to the aerosol container by the manual raising of cocking lever 22. However, when the device is locked-out by the control means the cocking force will not facilitate actuation of the valve since movement of the vane of the triggering mechanism is blocked by lever 24.

When doses are available to the patient, the control means causes operation of the solenoid 26, causing lever 28 to be pushed forwards and its hooked end to engage within an aperture in lever 24. When cocking lever 22 is then raised, levers 28 and 24 are also raised, so freeing the triggering mechanism for operation. Inhalation will then move the vane, freeing the nozzle block and allowing the cocking force to move the container relative to the valve, thereby firing the aerosol valve and delivering a dose of medicament.

The electronic circuitry is illustrated in diagrammatical form in Figure 2 and comprises an integrated circuit incorporating a clock regulated by a separate quartz crystal oscillator. The bi-stable d.c. solenoid is controlled via a power switch comprising field effect transistors (F.E.T's). The circuit includes a control switch for selecting the control period 'T', the dosage period 't' and the number of doses 'N'. A liquid crystal display is provided to display one or more types of information defined herein before. The electronic circuitry may readily be reduced to a chip and printed circuit board for accommodation within the device as shown at 13. A battery provides the necessary power. The liquid crystal display may be positioned at any suitable place on the housing.

The entire inhalation device may be compacted and the outer dimensions of the housing may be of the order of 90 mm x 60 mm x 30 mm.

The inhaler may be provided in either a disposable or re-usable form. In the former case the device is completely sealed to prevent access to the aerosol canister. In the latter, it is openable but has a locking mechanism to prevent unauthorised opening. This lock may be either mechanical or take the form of a code-operated electronic lock integrated with the device's electronic circuitry.

The invention will now be illustrated by the following examples.

The fentanyl citrate employed in the Examples contained 64% of the anhydrous base.

Example 1

The following formulations were prepared:

| | g/can | g/can |
|---|---|---|
| Fentanyl citrate | 0.0031 | 0.0031 |
| Span® 85 | 0.0069 (0.1%) | 0.0343 (0.5%) |
| Ethanol (21%w/w) | 1.4417 | 1.4143 |
| Propellant 12 | 5.4183 | 5.4183 |
| Total | 6.87 | 6.87 |

| | | |
|---|---|---|
| Fentanyl citrate | 0.075 | 0.075 |
| Span® 85 | 0.658 | - |
| Epikuron® 200 | - | 0.600 |
| Propellant 11 | 7.504 | 7.504 |
| Propellant 12 | 24.714 | 24.773 |
| Total | 32.951 | 32.952 |

All formulations were filled into glass bottles equipped with non-metering valves. The formulation containing Epikuron® 200 formed a solution.

Example 2

The following suspension formulations were prepared:

| | 0.5%Span® | 0.4%Span® | 0.2%Span® | 0.1%Span® |
|---|---|---|---|---|
| | g/can | g/can | g/can | g/can |
| Fentanyl citrate | 0.0748 | 0.0748 | 0.0748 | 0.0748 |
| Span® 85 | 0.1648 | 0.1318 | 0.0659 | 0.0330 |
| Propellant 11 | 7.9998 | 8.0314 | 8.0973 | 8.1302 |
| Propellant 12 | 24.7140 | 24.7140 | 24.7140 | 24.7140 |
| Total | 32.9534 | 32.952 | 32.952 | 32.952 |

The formulations were filled into glass bottles fitted with non-metering valves. Suspension formulations having a high Span® content exhibited a reduced tendency to agglomerate compared with formulations having a lower Span® content.

Example 3

The following suspension formulation was prepared:

| | g/can |
|---|---|
| Fentanyl citrate | 0.0187 |
| Span® 85 | 0.0412 |
| Propellant 11 | 1.9996 |
| Propellant 12 | 6.1785 |
| Total | 8.2380 |

The formulation was filled into 5ml plain aluminium vials fitted with 50 µl valves using a 2 stage pressure fitting

in which the non-volatile components were admixed and introduced into the vial, the valve crimped in place and the volatile propellant introduced into the vial through the valve under pressure.
Dosage can be varied by varying the valve size as follows:

| Valve Size | Dose Per Shot |
|---|---|
| 25μl | 50μg |
| 50μl | 100μg |
| 63μl | 126μg |
| 100μl | 200μg |

Example 4

Formulations were calculated for a delivery of 100μg of base per shot when a 50μl valve was fitted, and for a fill weight of 16g. The suspensions were filled into polyethylene terephthalate bottles to allow the appearance of the gross formulation to be studied. All units were prepared by two-stage pressure filling. The following stable suspensions contained Propellant 134a and ethanol in a weight ratio of 90 : 10.

0.1% w/w Span®85

| | mg/ml |
|---|---|
| Fentanyl citrate | 3.1250 |
| Span®85 | 1.1610 |
| Ethanol | 115.6714 |
| Propellant 134a | 1041.0426 |
| Total | 1161.0000 |

0.3% w/w Span®85

| | mg/ml |
|---|---|
| Fentanyl citrate | 3.1250 |
| Span®85 | 3.4830 |
| Ethanol | 115.4392 |
| Propellant 134a | 1038.9528 |
| Total | 1161.0000 |

0.5% w/w Span®85

| | mg/ml |
|---|---|
| Fentanyl citrate | 3.1250 |
| Span®85 | 5.8050 |
| Ethanol | 115.2070 |
| Propellant 134a | 1036.8630 |
| Total | 1161.0000 |

0.01% w/w Span® 85

| | mg/ml |
|---|---|
| Fentanyl citrate | 3.1250 |
| Span® 85 | 0.1161 |
| Ethanol | 115.7759 |
| Propellant 134a | 1041.9830 |
| Total | 1161.0000 |

0.02% w/w Span® 85

| | mg/ml |
|---|---|
| Fentanyl citrate | 3.1250 |
| Span® 85 | 0.2322 |
| Ethanol | 115.7643 |
| Propellant 134a | 1041.8785 |
| Total | 1161.0000 |

0.05% w/w Span® 85

| | mg/ml |
|---|---|
| Fentanyl citrate | 3.1250 |
| Span® 85 | 0.5805 |
| Ethanol | 115.7294 |
| Propellant 134a | 1041.5651 |
| Total | 1161.0000 |

Example 5

The following stable suspensions were prepared as in Example 4 and contained Propellant 134a and n-Pentane in a ratio of 90 : 10.

0.1% w/w Span® 85

| | mg/ml |
|---|---|
| Fentanyl citrate | 3.1250 |
| Span® 85 | 1.1610 |
| Pentane | 115.6714 |
| Propellant 134a | 1041.0426 |
| Total | 1161.0000 |

0.3% w/w Span® 85

| | mg/ml |
|---|---|
| Fentanyl citrate | 3.1250 |
| Span® 85 | 3.4830 |
| Pentane | 115.4392 |
| Propellant 134a | 1038.9528 |
| Total | 1161.0000 |

0.5% w/w Span® 85

| | mg/ml |
|---|---|
| Fentanyl citrate | 3.1250 |
| Span® 85 | 5.8050 |
| Pentane | 115.2070 |
| Propellant 134a | 1036.8630 |
| Total | 1161.0000 |

Example 6

The following suspensions were prepared as in Example 4 and contained Propellant 134a and Propellant 11 in a ration of 95 : 5.

0.1% w/w Span® 85

| | mg/ml |
|---|---|
| Fentanyl citrate | 3.1250 |
| Span® 85 | 1.1610 |
| Propellant 11 | 57.8357 |
| Propellant 134a | 1098.8783 |
| Total | 1161.0000 |

0.2% w/w Span® 85

| | mg/ml |
|---|---|
| Fentanyl citrate | 3.1250 |
| Span® 85 | 2.3220 |
| Propellant 11 | 57.7776 |
| Propellant 134a | 1097.7754 |
| Total | 1161.0000 |

Example 7

Fentanyl citrate formulations of the invention were compared with morphine sulphate, morphine base and diamorphine hydrochloride formulations presented as metered-dose inhalation aerosols in various single-dose and repeat-dose experiments in rodent and non-rodent species.
The comparative formulations were as follows.

| | g/can |
|---|---|
| Diamorphine HCℓ | 0.063 (10% drug overage) |
| Span® 85 (20% of drug) | 0.013 |
| Propellant 11 | 1.640 |
| Propellant 12 | 5.145 |
| Total | 6.860 |
| | |
| Morphine Sulphate | 0.147 |
| Span® 85 | 0.034 |
| Propellant 11 | 1.534 |
| Propellant 12 | 5.145 |
| Total | 6.860 |
| | |
| Morphine base | 0.1200 |
| Span® 85 | 0.0412 |
| Propellant 11 | 1.8983 |
| Propellant 12 | 6.1785 |
| Total | 8.2380 |

Each formulation was filled into 5ml vials equipped with 50µl dispensing valves.

Rodent Inhalation Exposure Procedure

The system consisted of an aluminium cylindrical chamber of approximately 41.5 litre volume. It was fitted with a flat top which supported an electrically driven mechanism, capable of actuating up to six inverted metered-dose inhaler (MDI) aerosol cans simultaneously. The duration and frequency of operation was controlled by a time switch.

The MDI can nozzles opened directly into the exposure chamber where the administered aerosol dose was mixed and dispersed by a regulated constant supply of clean dry air. An extract duct at the base of the chamber was connected via a filter to a vacuum system. The pressure within the chamber was maintained just below atmospheric pressure.

MDI cans were selected, weighed and inverted in the template of the actuating mechanism. The ram mechanism was positioned to cover the bases of the cans so that each downward stroke of the ram would actuate the cans causing them to fire simultaneously into the exposure chamber.

Each rodent was held in an individual, tapered, polycarbonate restraint tube, fitted onto the exposure chamber and sealed by means of a push fit through a rubber 'O' ring. Only the animal's nose was exposed to the test atmosphere.

Animals were observed both during and following dosing for clinical signs of drug effect.

Non-Rodent Inhalation Exposure Procedure

Inhalation dosing to Beagle dogs was performed using a dosing apparatus, comprising a mouthpiece, face

mask, and modified MDI. The mouthpiece was located inside the animal's mouth on top of the tongue, and the face mask, incorporating the mouthpiece, sealed around the dog's snout by means of a rubber sleeve. The mask was connected via a one-way flap valve and exhaust tube to an extract system. The modified MDI was attached by a push fit connection to the distal end of the mouthpiece.

When assembled and fitted to the dog the animals' respiratory cycle was clearly indicated by the movement of the one-way flap valve. The MDI can was actuated manually in the normal way, by downward pressure at a time to coincide with inspiration.

Non-Rodent Intranasal Exposure Procedure

Animals were dosed by direct application of the test material into each nostril by means of an adaptor fitted to the MDI.

Results

Rodents

Fentanyl citrate in formulation of Example 3

Single Exposure Period

Using six cans firing at six shots per minute for a single-exposure period between three and ten minutes, the animals showed gradual sedation and eventually exhibited marked narcosis by ten minutes.

Multiple exposure period

Using six cans firing at six shots per minute for three ten minute exposures daily for up to five days, marked narcosis or sedation was observed for all animals for up to two hours post exposure (see Table 1).

Diamorphine hydrochloride

Using six cans firing at six shots per minute for a single-exposure period between ten and fifteen minutes, no overt pharmacological effects were noted. Animals showed a slight reduction in respiratory rate during exposure and slight salivation following exposure for a short time. Post exposure animals were slightly subdued.

Morphine sulphate

Animals were exposed to an atmosphere generated from six cans actuated at a rate of six shots per minute for a period of ten or sixty minutes. There was no observable effect seen in the behaviour of the animals following the ten minute exposure. During the second exposure, the rate of respiration dropped and following exposure the animals appeared to be very slightly subdued and easily startled.

Non-Rodent

Fentanyl citrate in Formulation of Example 3

Single exposure

Administration of test material was performed, by the inhalation and intranasal routes. Doses administered ranged from 8 to 25 actuations per session for the inhalation administration and from 5 to 35 actuations per session for the intranasal route of administration. Following inhalation, fentanyl citrate caused rapid and marked sedation with the effect lasting for at least one hour at high doses. Similar observations were seen following intranasal administration.

Multiple-exposure period

Animals were treated with a nominal dose of 0.13mg/kg fentanyl three times daily over a five day period. To achieve the correct dose, animals were weighed daily, and the body weight used to calculate the requisite

number of metered dose aerosol actuations to be administered. (Results are shown in Table 2).

Diamorphine hvdrochloride

A nominal dose of 30mg diamorphine hydrochloride administered by the inhalation route caused sedation in 1 out of 2 dogs. However, subsequent administration of the high dose formulation at similar or higher nominal doses, failed to produce signs of sedation in either animal. In subsequent experiments a nominal dose of 8 - 9 mg/kg diamorphine hydrochloride administered intranasally, was capable of producing a marked degree of sedation in dogs whilst a nominal dose of 10 - 13 mg/kg administered over three sessions caused a constant state of sedation throughout the whole dosing period.

Morphine base

Animals appeared normal following inhalation of a nominal dose of 6.5 mg/kg. Clear signs of sedation were noted after a nominal dose of 13.0 mg/kg was administered. These signs persisted for at least two hours. No drug related effects were seen following intranasal administration of 225 actuations over a 35 minute period.

Morphine sulphate

A total daily dose of 450 actuations of the formulation were administered by the intranasal route. Minimal drug-related clinical signs were observed during and following treatment. Up to 330 actuations of a higher concentration formulation were also administered intranasally. At the higher dose, animals exhibited a reduced pupillary constriction on reflex to a light stimulus, became subdued and slightly sedated.

Fentanyl citrate has been demonstrated in two animal models to be a highly potent, quick acting compound with pharmacological actions persisting for about two hours. These properties make it a good candidate for developing into a patient controlled analgesia dosage form for administration via the inhalation route. Morphine base and Morphine sulphate preparations did not show a suitable rapid onset of action, and were considerably less potent by the inhalation route than might have been expected from the intravenous dosing data.

TABLE 1

Fentanyl Citrate: Inhalation Tolerance Study in Rats - Clinical Signs

| Clinical Signs Observed | | Day/Dose Session/Number of Animals Affected | | | | | | | | | | | | | | |
|---|---|---|---|---|---|---|---|---|---|---|---|---|---|---|---|---|
| | | Day 1 | | | Day 2 | | | Day 3 | | | Day 4 | | | Day 5 | | |
| | | 1 | 2 | 3 | 1 | 2 | 3 | 1 | 2 | 3 | 1 | 2 | 3 | 1 | 2 | 3 |
| During Exposure: | | | | | | | | | | | | | | | | |
| Reduction in Respiratory Rate | | 5M/5F | 5M/5F | 5M/5F | 5M/5F | 5M/5F | 5M/5F | 5M/5F | 5M/5F | 5M/5F | 5M/5F | 5M/5F | 5M/5F | 5M/5F | 5M/5F | 5M/5F |
| Post Exposure: | | | | | | | | | | | | | | | | |
| + 1 min: | Narcosis | 5M/5F | 5M/5F | 5M/5F | 5M/5F | 5M/5F | 5M/5F | 5M/5F | 4M/4F | 5M/5F | 5M/5F | 5M/5F | 4M/3F | 5M/5F | 5M/4F | 3M/2F |
| | Sedated | - | - | - | - | - | - | - | 1M/1F | - | - | - | - | - | -/1F | 2M/3F |
| +30 min: | Narcosis | 5M/5F | 5M/5F | 5M/5F | 3M/5F | 5M/5F | -/1F | 5M/5F | 1M/2F | - | 2M/1F | 1M/1F | 1M/2F | 2M/2F | 2M/1F | 1M/1F |
| | Sedated | - | - | - | 2M/- | - | 5M/4F | - | 4M/3F | 5M/5F | 3M/4F | 4M/4F | 4M/3F | 3M/3F | 3M/4F | 4M/4F |
| + 1 h : | Narcosis | 5M/5F | 5M/5F | 5M/5F | 1M/1F | 1M/2F | - | - | - | - | 1M/1F | - | - | 1M/1F | - | - |
| | Sedated | - | - | - | 4M/4F | 4M/3F | 5M/5F | 5M/5F | 5M/5F | 5M/5F | 4M/4F | 5M/5F | 5M/5F | 4M/4F | 5M/5F | 5M/5F |
| + 2 h : | Narcosis | 5M/5F | 1M/2F | - | 1M/1F | - | - | - | - | - | - | - | - | - | - | - |
| | Sedated | - | 4M/3F | 5M/5F | 4M/4F | 5M/5F | 5M/5F | 5M/5F | 5M/5F | 5M/5F | 5M/5F | 5M/5F | 5M/5F | 5M/5F | 5M/5F | 5M/5F |
| Aggressive behaviour noted when animals returned to group cage at end of each dosing day. This lasted for approximately 30 minutes. | | | | | | | | | | | | | | | | |

Note: to facilitate ease of clinical signs observation, the animals were placed singly in cages following each dose session.

min = minutes  h = hours  Narcosis = unconscious  Sedated = reduced activity  M = male  F = female

TABLE 2

Fentanyl Citrate: Inhalation Tolerance Study in Dogs - Clinical Signs

| Clinical Signs Observed | Day/Dose Session | | | | | | | | | | | | | | |
|---|---|---|---|---|---|---|---|---|---|---|---|---|---|---|---|
| | Day 1 | | | Day 2 | | | Day 3 | | | Day 4 | | | Day 5 | | |
| | 1 | 2 | 3 | 1 | 2 | 3 | 1 | 2 | 3 | 1 | 2 | 3 | 1 | 2 | 3 |
| **Predose:** | | | | | | | | | | | | | | | |
| NAD | M F | M F | M F | M F | M F | M F | M F | M F | M F | M F | M F | M F | M F | M F | M F |
| Sedated | - - | - - | - - | - - | - - | - - | - - | - - | - - | - - | - - | - - | - - | - - | - - |
| **During Dosing:** | | | | | | | | | | | | | | | |
| NAD | - - | M - | M F | - - | - - | M F | M F | M F | M F | - - | M - | M - | M - | M F | M F |
| Slightly sedated | M - | - - | - - | - - | - - | - - | - - | - - | - - | - - | - - | - - | - - | - - | - - |
| Ataxic | M - | - - | - - | - - | - - | - - | - - | - - | - - | - - | - - | - - | - - | - - | - - |
| Tending to sit | - - | - - | - - | - - | - - | - - | - - | - - | - - | - - | - - | - - | - - | - - | - - |
| Salivation | M F | - F | - - | M F | M F | - - | - - | - - | - - | M F | - F | - F | - F | - - | - - |
| Vocalising | - F | - - | - - | - - | - - | - - | - - | - - | - - | - - | - - | - - | - - | - - | - - |
| Heart Rate (I/D) | - - | - - | - - | - - | - - | - - | - - | - - | - - | - - | - - | - - | - - | - - | - - |
| Respiration (I/D) | - - | - - | - - | - - | - - | - - | - - | - - | - - | - - | - - | - - | - - | - - | - - |
| **Post Dose:** | | | | | | | | | | | | | | | |
| Tending to sit | - F | - F | - F | M F | M F | - F | - F | - F | - - | - F | - F | - - | - - | - - | - - |
| Crouched walk | - - | - - | - F | - - | - - | - F | - - | - F | - - | - - | - - | - F | - - | - - | - F |
| Urination/ defaecation | - - | - F | - - | - - | - - | - - | - - | - - | - - | - - | - - | - - | - - | - - | - - |
| Salivation | - - | - - | - - | - - | - - | - - | M F | M - | - - | M - | - - | - - | M F | - F | M F |
| Ataxic | M - | M - | - F | M - | M - | M - | M - | M - | M - | M - | - - | M - | - - | - - | M F |
| Sedated | M - | M - | M F | M - | M - | M - | M - | - F | M - | M - | M - | M - | M - | M - | M F |
| Vocalising | - F | M F | M F | M F | M F | M F | M F | M F | M F | M F | M F | M F | - - | - F | - F |
| Pinpoint pupil | - - | - - | - - | - - | - - | - - | - - | - - | - - | - - | - - | - - | M - | M - | - - |
| Heart rate (I/D) | - FD | MD - | MD - | MD FD | MD FD | MD - | - - | - - | MD - | MD - | MD - | MD - | - - | - - | - - |
| Respiration I/D) | - FI | MD - | MD FI | MD FI | MD - | - FI | MD - | - - | - - | MD - | - - | - - | - FI | - - | - - |

I = Increased D = Decreased M = Male F = Female

Note: both animals generally recovered ca 1 hour after each dose session

## Claims

1. An aerosol formulation comprising fentanyl or a physiologically acceptable derivative thereof dispersed or dissolved in an aerosol propellant.

2. An aerosol formulation as claimed in Claim 1 comprising fentanyl citrate.

3. An aerosol formulation as claimed in Claim 1 or Claim 2 in which fentanyl or a physiologically acceptable derivative thereof is present in an amount of from 0.05% to 5.0% by weight of the total composition.

4. An aerosol formulation as claimed in any preceding claim which additionally comprises a solvent for fentanyl in an amount within the range from 5 to 25% by weight based on the total composition.

5. An aerosol formulation as claimed in any preceding claim which additionally comprises a surface active agent, in which the weight ratio of surface active agent to fentanyl is in the range 1 : 100 to 10 : 1.

6. An aerosol formulation as claimed in Claim 5 in which the surface active agent is selected from sorbitan trioleate, oleic acid, lecithin and fluorinated surfactants.

7. An aerosol formulation as claimed in any preceding claim which comprises one or more of the following propellants at a concentration within the following range:
   from 15 to 25% by weight trichloromonofluoromethane,
   from 50 to 90% by weight dichlorodifluoromethane, and
   from 5 to 50% by weight monochlorodifluoromethane.

8. An aerosol composition as claimed in any one of Claims 1 to 6 in which the aerosol propellant comprises 1,1,1,2-tetrafluoroethane, which is present in an amount of at least 50% by weight of the formulation.

9. An aerosol composition as claimed in Claim 8 which comprises 1,1,1,2-tetrafluoroethane, a surface active agent and at least one adjuvant having a polarity higher than 1,1,1,2-tetrafluoroethane, in which the weight ratio of 1,1,1,2-tetrafluoroethane: compound of higher polarity is in the range 50 : 50 to 99 : 1.

10. An aerosol formulation as claimed in Claim 9 in which the compound having a higher polarity is selected from ethyl alcohol, isopropyl alcohol, n-pentane, isopentane, neopentane, and mixtures thereof.

11. An aerosol composition as claimed in Claim 8 in which the fentanyl or physiologically acceptable derivative thereof is in the form of a finely divided solid material coated with a surface-active dispersing agent which constitutes at least 0.001% by weight of the coated solid material, and is suspended in an aerosol propellant comprising 1,1,1,2-tetrafluoroethane and optionally an adjuvant having a polarity equal to or less than that of 1,1,1,2-tetrafluoroethane.

12. An aerosol formulation as claimed in Claim 11 in which the finely-divided solid material constitutes up to 10.0 percent by weight of the total composition and the surface-active dispersing agent constitutes between 0.01 and 1.0 percent by weight of the finely-divided solid material.

13. An aerosol formulation as claimed in Claim 11 or Claim 12 in which the surface-active dispersing agent is selected from perfluorinated sulfonamide alcohol phosphate esters and their salts; perfluorinated alcohol phosphate ester free acids and their salts; perfluorinated alkyl sulfonamide alkylene quaternary ammonium salts; N,N-(carboxyl-up to $C_4$ substituted alkyl) perfluorinated alkyl sulfonamides and their salts; and mixtures thereof.

14. An aerosol formulation as claimed in any one of Claims 11 to 13 in which the propellant system comprises a mixture of 1,1,1,2-tetrafluoroethane and an adjuvant selected from perfluoropropane, perfluorobutane, octafluorocyclobutane, perfluoropentane, perfluorohexane, perfluorotributylamine, perfluoromethylcyclohexane and perfluorodecalin.

15. A pressurised aerosol inhaler comprising a container containing an aerosol formulation as claimed in any preceding claim and a valve capable of dispensing doses of formulation.

16. A pressurised aerosol inhaler as claimed in Claim 15 additionally comprising control means to control the

dosing frequency from the valve.

17. A pressurised aerosol inhaler as claimed in Claim 16 in which the control means is constructed and arranged such that not more than a predetermined maximum number of doses may be dispensed within a set period of time.

**Patentansprüche**

1. Aerosolformulierung, umfassend Fentanyl oder ein physiologisch verträgliches Derivat davon, das in einem Aerosoltreibmittel dispergiert oder gelöst ist.

2. Aerosolformulierung nach Anspruch 1, umfassend Fentanylcitrat.

3. Aerosolformulierung nach Anspruch 1 oder 2, wobei Fentanyl oder ein physiologisch verträgliches Derivat davon in einer Menge von 0,05 bis 5,0 Gew.-%, bezogen auf die Gesamtzusammensetzung vorhanden ist.

4. Aerosolformulierung nach einem der vorstehenden Ansprüche, die zusätzlich ein Lösungsmittel für Fentanyl in einer Menge im Bereich von 5 bis 25 Gew.-%, bezogen auf die Gesamtzusammensetzung, umfaßt.

5. Aerosolformulierung nach einem der vorstehenden Ansprüche, die zusätzlich ein oberflächenaktives Mittel umfaßt, wobei das Gewichtsverhältnis des Tensids zu Fentanyl im Bereich von 1 : 100 bis 10 : 1 ist.

6. Aerosolformulierung nach Anspruch 5, wobei das oberflächenaktive Mittel ausgewählt ist aus Sorbitantrioleat, Ölsäure, Lecithin und fluorierten oberflächenaktiven Mitteln.

7. Aerosolformulierung nach einem der vorstehenden Ansprüche, umfassend eines oder mehrere der folgenden Treibmittel in einer Konzentration im angegebenen Bereich:
15 bis 25 Gew.-% Trichlormonofluormethan,
50 bis 90 Gew.-% Dichlordifluormethan und
5 bis 50 Gew.-% Monochlordifluormethan.

8. Aerosolformulierung nach einem der Ansprüche 1 bis 6, wobei das Aerosoltreibmittel 1,1,1,2-Tetrafluorethan in einer Menge von mindestens 50 Gew.-%, bezogen auf die Formulierung, umfaßt.

9. Aerosolformulierung nach Anspruch 8, die 1,1,1,2-Tetrafluorethan, ein oberflächenaktives Mittel und mindestens ein Adjuvans mit einer höheren Polarität als 1,1,1,2-Tetrafluorethan umfaßt, wobei das Gewichtsverhältnis von 1,1,1,2-Tetrafluorethan zu der Verbindung mit der höheren Polarität im Bereich von 50 : 50 bis 99 : 1 liegt.

10. Aerosolformulierung nach Anspruch 9, wobei die Verbindung mit einer höheren Polarität ausgewählt ist aus Ethanol, Isopropanol, n-Pentan, Isopentan, Neopentan und Gemischen davon.

11. Aerosolformulierung nach Anspruch 8, wobei das Fentanyl oder das physiologisch verträgliche Derivat davon in Form eines mit einem oberflächenaktiven Dispersionsmittel beschichteten feinverteilten, festen Materials vorliegt, wobei das Dispersionsmittel mindestens 0,001 Gew.-% des beschichteten, festen Materials ausmacht, und in einem Aerosoltreibmittel, umfassend 1,1,1,2-Tetrafluorethan und gegebenenfalls einem Adjuvans mit einer Polarität, die gleich oder geringer ist als die von 1,1,1,2-Tetrafluorethan suspendiert ist.

12. Aerosolformulierung nach Anspruch 11, wobei das feinverteilte, feste Material bis zu 10 Gew.-% der Gesamtzusammensetzung ausmacht und das oberflächenaktive Dispersionsmittel zwischen 0,01 und 1,0 Gew.-% des feinverteilten, festen Materials ausmacht.

13. Aerosolformulierung nach Anspruch 11 oder 12, wobei das oberflächenaktive Dispersionsmittel ausgewählt ist aus perfluorierten Sulfonamidalkoholphosphatestern und deren Salzen, perfluorierten alkoholphosphatesterfreien Säuren und deren Salzen, perfluorierten alkylsulfonamidalkylenquaternären Ammoniumsalzen, N,N-(Carboxyl-substituierten-bis zu $C_4$-Alkyl)perfluorierten Alkylsulfonamiden und deren

Salzen und Gemischen davon.

**14.** Aerosolformulierung nach einem der Ansprüche 11 bis 13, wobei das Treibmittel ein Gemisch aus 1,1,1,2-Tetrafluorethan und einem Adjuvans, ausgewählt aus Perfluorpropan, Perfluorbutan, Octafluorcyclobutan, Perfluorpentan, Perfluorhexan, Perfluortributylamin, Perfluormethylcyclohexan und Perfluordecalin umfaßt.

**15.** Unter Druck stehender Aerosolinhalator, umfassend einen Behälter, enthaltend eine Aerosolformulierung nach einem der vorstehenden Ansprüche und ein Ventil, das fähig ist, Dosierungen der Formulierung auszugeben.

**16.** Unter Druck stehender Aerosolinhalator nach Anspruch 15, der zusätzlich eine Regelvorrichtung zur Regelung der Frequenz, mit der von dem Ventil Dosierungen ausgegeben werden, umfaßt.

**17.** Unter Druck stehender Aerosolinhalator nach Anspruch 16, wobei die Regelvorrichtung derart beschaffen und angeordnet ist, daß nicht mehr als eine vorbestimmte Maximalzahl von Dosierungen während einer vorgegebenen Zeitdauer ausgegeben werden können.

## Revendications

**1.** Formulation d'aérosol comprenant du fentanyle ou un de ses dérivés physiologiquement acceptables, dispersé ou dissous dans un agent propulseur d'aérosol.

**2.** Formulation d'aérosol suivant la revendication 1, comprenant du citrate de fentanyle.

**3.** Formulation d'aérosol suivant la revendication 1 ou la revendication 2, dans laquelle du fentanyle ou un de ses dérivés physiologiquement acceptables est présent en une quantité de 0,05 % à 5,0 % en poids de la composition totale.

**4.** Formulation d'aérosol suivant l'une quelconque des revendications précédentes, qui comprend en outre un solvant du fentanyle en une quantité comprise dans l'intervalle de 5 à 25 % en poids sur la base de la composition totale.

**5.** Formulation d'aérosol suivant l'une quelconque des revendications précédentes, qui comprend en outre un agent tensio-actif, le rapport pondéral de l'agent tensio-actif au fentanyle étant compris dans l'intervalle de 1:100 à 10:1.

**6.** Formulation d'aérosol suivant la revendication 5, dans laquelle l'agent tensio-actif est choisi entre le trioléate de sorbitanne, l'acide oléique, la lécithine et des surfactants fluorés.

**7.** Formulation d'aérosol suivant l'une quelconque des revendications précédentes, qui comprend un ou plusieurs des agents propulseurs suivants à une concentration comprise dans les intervalles suivants :
15 à 25 % en poids de trichloromonofluorométhane,
50 à 90 % en poids de dichlorodifluorométhane, et
5 à 50 % en poids de monochlorodifluorométhane.

**8.** Composition d'aérosol suivant l'une quelconque des revendications 1 à 6, dans laquelle l'agent propulseur d'aérosol comprend du 1,1,1,2-tétrafluoréthane, qui est présent en une quantité d'au moins 50 % en poids de la formulation.

**9.** Composition d'aérosol suivant la revendication 8, qui comprend du 1,1,1,2-tétrafluoréthane, un agent tensio-actif et au moins un adjuvant ayant une polarité supérieure à celle du 1,1,1,2-tétrafluoréthane, le rapport pondéral du 1,1,1,2-tétrafluoréthane au composé de plus forte polarité étant compris dans l'intervalle de 50:50 à 99:1.

**10.** Formulation d'aérosol suivant la revendication 9, dans laquelle le composé ayant une plus forte polarité est choisi entre l'alcool éthylique, l'alcool isopropylique, le n-pentane, l'isopentane, le néopentane et leurs mélanges.

11. Composition d'aérosol suivant la revendication 8, dans laquelle le fentanyle ou son dérivé physiologiquement acceptable est sous forme d'une substance solide finement divisée enrobée avec un agent dispersant tensio-actif qui représente au moins 0,001 % en poids de la substance solide enrobée, et est mis en suspension dans un agent propulseur d'aérosol comprenant du 1,1,1,2-tétrafluoréthane et, facultativement, un adjuvant ayant une polarité égale ou inférieure à celle du 1,1,1,2-tétrafluoréthane.

12. Formulation d'aérosol suivant la revendication 11, dans laquelle la substance solide finement divisée représente jusqu'à 10,0 % en poids de la composition totale et l'agent dispersant tensio-actif représente 0,01 à 1,0 % en poids de la substance solide finement divisée.

13. Formulation d'aérosol suivant la revendication 11 ou la revendication 12, dans laquelle l'agent dispersant tensio-actif est choisi entre des esters phosphoriques de sulfamide-alcools perfluorés et leurs sels ; des acides libres d'esters phosphoriques d'alcools perfluorés et leurs sels ; des sels d'alkylène-ammonium quaternaire d'alkylsulfamides perfluorés ; des N,N-(alkyle jusqu'à $C_4$ à substituant carboxyle)-alkylsulfamides perfluorés et leurs sels ; et leurs mélanges.

14. Formulation d'aérosol suivant l'une quelconque des revendications 11 à 13, dans laquelle l'agent propulseur comprend un mélange de 1,1,1,2-tétrafluoréthane et d'un adjuvant choisi entre le perfluoropropane, le perfluorobutane, l'octafluorocyclobutane, le perfluoropentane, le perfluorohexane, la perfluorotributylamine, le perfluorométhylcyclohexane et la perfluorodécaline.

15. Inhalateur d'aérosol pressurisé comprenant un récipient contenant une formulation d'aérosol suivant l'une quelconque des revendications précédentes et une valve capable de distribuer des doses de formulation.

16. Inhalateur d'aérosol pressurisé suivant la revendication 15, comprenant en outre un moyen de régulation pour ajuster la fréquence d'administration à partir de la valve.

17. Inhalateur d'aérosol pressurisé suivant la revendication 16, dans lequel le moyen de régulation présente une structure et une disposition telles qu'un nombre non supérieur à un nombre maximal prédéterminé de doses puisse être distribué en un temps déterminé.

Fig.1.

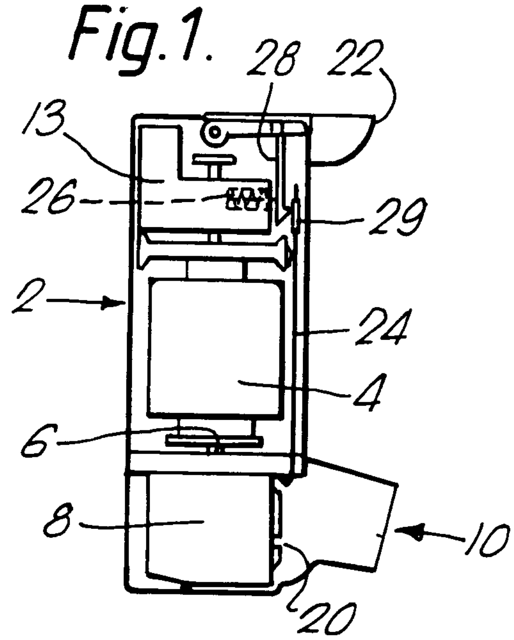
28
22
13
26
29
2
24
4
6
8
10
20

Fig.2.

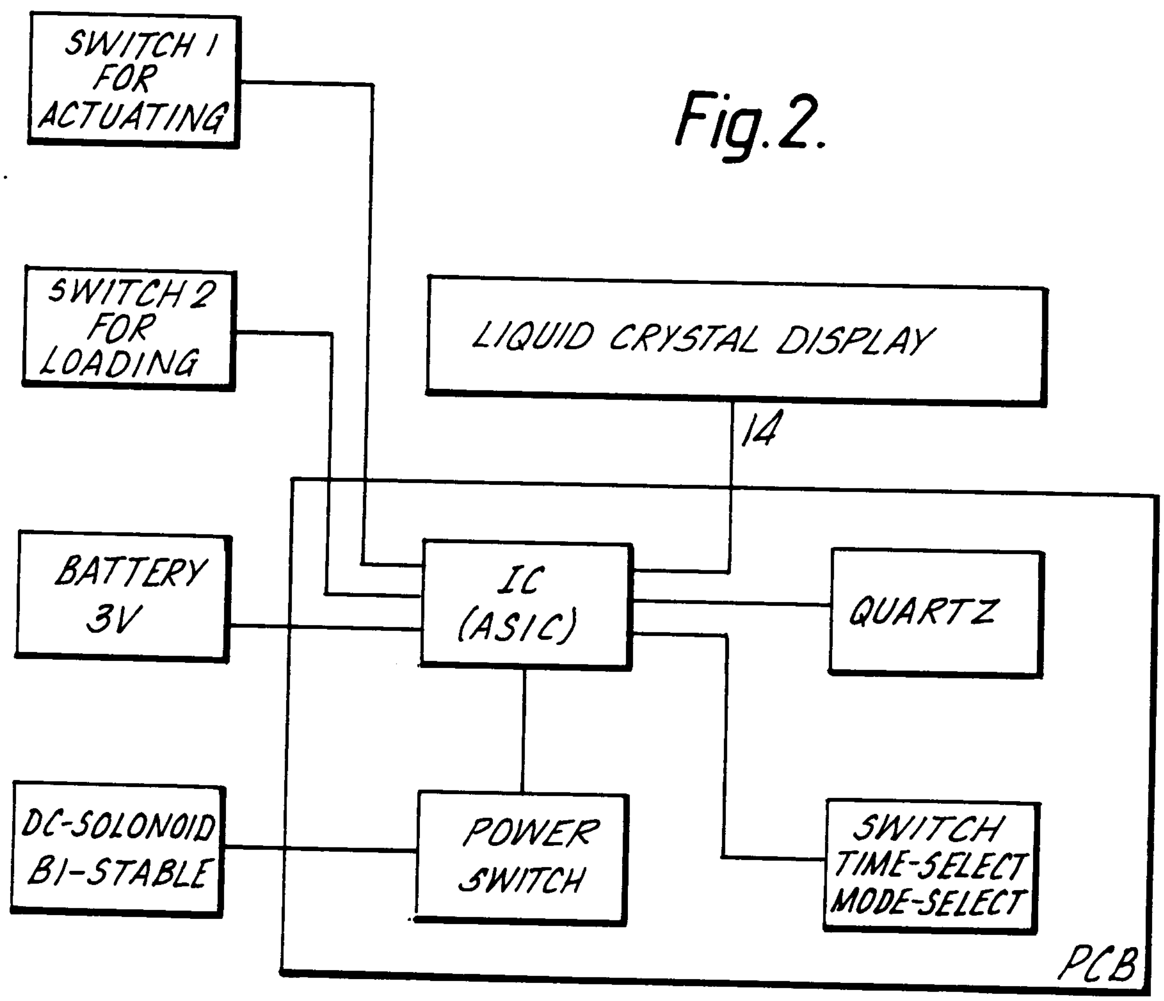
SWITCH 1 FOR ACTUATING
SWITCH 2 FOR LOADING
LIQUID CRYSTAL DISPLAY
14
BATTERY 3V
IC (ASIC)
QUARTZ
DC-SOLONOID BI-STABLE
POWER SWITCH
SWITCH TIME-SELECT MODE-SELECT
PCB